(19) **Europäisches Patentamt / European Patent Office / Office européen des brevets**

(11) **EP 1 513 444 B1**

(12) **FASCICULE DE BREVET EUROPEEN**

(45) Date de publication et mention
de la délivrance du brevet:
**18.03.2015 Bulletin 2015/12**

(21) Numéro de dépôt: **03740631.1**

(22) Date de dépôt: **16.04.2003**

(51) Int Cl.:
*A61B 5/04* *(2006.01)*    *A61B 5/0468* *(2006.01)*

(86) Numéro de dépôt international:
**PCT/FR2003/001226**

(87) Numéro de publication internationale:
**WO 2003/101291 (11.12.2003 Gazette 2003/50)**

(54) **TRAITEMENT FREQUENTIEL D'UNE SERIE RR DANS UN SIGNAL CARDIAQUE ANALOGIQUE**

FREQUENZVERARBEITUNG EINER RR-SERIE EINES ANALOGEN HERZSIGNALS

FREQUENCY PROCESSING OF AN RR SERIES IN AN ANALOGUE CARDIAC SIGNAL

(84) Etats contractants désignés:
**AT BE BG CH CY CZ DE DK EE ES FI FR GB GR HU IE IT LI LU MC NL PT RO SE SI SK TR**

(30) Priorité: **31.05.2002 FR 0206676**

(43) Date de publication de la demande:
**16.03.2005 Bulletin 2005/11**

(73) Titulaire: **CENTRE HOSPITALIER REGIONAL ET UNIVERSITAIRE DE LILLE**
**59037 Lille Cédex (FR)**

(72) Inventeurs:
• **LOGIER, Régis**
  **F-59700 Marcq en Baroeul (FR)**
• **DASSONNEVILLE, Alain**
  **F-59116 Houplines (FR)**

(74) Mandataire: **Matkowska, Franck**
**Matkowska & Associés**
**9, rue Jacques Prévert**
**59650 Villeneuve d'Ascq (FR)**

(56) Documents cités:
**US-A- 5 042 499    US-A- 5 755 671**
**US-B1- 6 330 469**

• **BIGGER J T ET AL: "RR VARIABILITY IN HEALTHY, MIDDLE-AGED PERSONS COMPARED WITH PATIENTS WITH CHRONIC CORONARY HEART DISEASE OR RECENT ACUTE MYOCARDIAL INFARCTION" CIRCULATION, AMERICAN HEART ASSOCIATION, DALLAS, TX, US, vol. 91, no. 7, 1 avril 1995 (1995-04-01), pages 1936-1943, XP000610690 ISSN: 0009-7322**

**Description**

La présente invention concerne d'une manière générale le domaine de l'analyse fréquentielle d'une série RR construite à partir d'un signal bio-électrique analogique, qui est caractéristique du rythme cardiaque d'un être vivant, et qui est désigné dans le présent texte par les termes *«signal cardiaque».* Cette analyse fréquentielle est réalisée dans le but d'extraire automatiquement de la série RR une ou plusieurs informations quantitatives qui permettent de caractériser l'activité du système nerveux autonome (SNA). Le signal cardiaque est de préférence, mais de manière non exclusive, un signal électrocardiographique (ECG), ou encore un signal cardiaque foetal mesuré au moyen d'une électrode placé sur le scalp d'un foetus ou au moyen d'un capteur ultrason placé sur l'abdomen maternel.

Dans ce domaine technique, l'invention a pour principaux objets une méthode de traitement fréquentiel d'une série RR ainsi qu'un procédé d'acquisition et de traitement électronique d'un signal cardiaque analogique utilisant ladite méthode de traitement fréquentiel. L'invention a également pour objet un système d'acquisition et de traitement en temps réel d'un signal cardiaque. Une des application préférentielle, mais non exclusive de l'invention, est la mesure et la surveillance de la souffrance foetale.

D'un point de vue physiologique, le coeur d'un être vivant, isolé de toute influence extérieure, se contracte automatiquement de façon très régulière comme un métronome, sous l'action du noeud sinusal qui génère un influx nerveux indépendant, et par là-même provoque une contraction spontanée du muscle cardiaque. Le coeur n'est toutefois pas isolé, mais est relié au Système Nerveux Autonome (SNA), par l'intermédiaire des systèmes parasympathique et sympathique. Ce système nerveux autonome influe sur l'activité du coeur : le système sympathique accélère le rythme cardiaque, tandis que le système parasympathique le ralentit. Ainsi, malgré une certaine autonomie, le coeur subit des influences du système nerveux autonome, ce qui permet notamment à l'organisme d'un être vivant d'adapter le rythme cardiaque en fonction de ses besoins, dans des limites toutefois raisonnables. On comprend en conséquence que l'analyse de l'évolution dans le temps du rythme cardiaque, et en particulier des variations du rythme cardiaque (variation des battements du coeur) permet d'obtenir une information importante sur l'activité du système cardiaque, et plus particulièrement sur l'activité du système nerveux autonome. Or la connaissance de l'activité du SNA peut être d'une aide précieuse dans l'élaboration d'un diagnostic de bon nombre de situations cliniques. Sur ce sujet, on pourra se référer par exemple à la publication ci-après :

Lacroix D, Logier R., Kacet S., Hazard J-R, Dagano J. (1992) : « Effects of consécutive administration of central and peripheral anticholinergic agents on respiratory sinus arrhythmia in normal subjects, J. of the Autonomic Nervous System", Vol 39, pages 211-218

Pour étudier ces fluctuations du rythme cardiaque, et par là-même l'activité du SNA, on a déjà depuis 1970 développé différentes techniques d'analyse spectrale d'un signal qui représente l'évolution dans le temps du rythme (ou fréquence) cardiaque instantané, et qui est obtenu après échantillonnage d'un signal bio-électrique analogique, caractéristique du rythme cardiaque d'un être vivant, et dit par la suite « signal cardiaque ».

Acquisition du signal cardiaque et construction d'une série (RR)

Pour acquérir ce signal cardiaque (analogique), différentes techniques d'acquisition invasives ou non invasives sont connues. Une technique invasive connue consiste par exemple à utiliser un capteur de pression sanglante relié à un cathéter introduit dans une artère. Parmi les méthodes non invasives connues, on trouve par exemple l'utilisation d'un capteur de pouls infrarouge, ou l'acquisition d'un signal électrocardiographique (ECG) au moyen d'un électrocardiographe. Egalement, dans le domaine de la surveillance foetale, il est connu d'utiliser un appareil, communément appelé cardiotocographe, qui permet l'enregistrement simultané des contractions utérines et des battements cardiaques foetaux, lesquels battement sont recueillis par une électrode placée sur le scalp foetal ou par un capteur à ultrasons à travers la paroi abdominale maternelle.

En dehors du domaine particulier de la surveillance foetale, la méthode d'acquisition d'un signal ECG est en pratique la plus couramment utilisée à ce jour, car outre son caractère non invasif, elle permet avantageusement d'obtenir un signal plus précis que celui obtenu par exemple au moyen d'un capteur de pouls infrarouge.

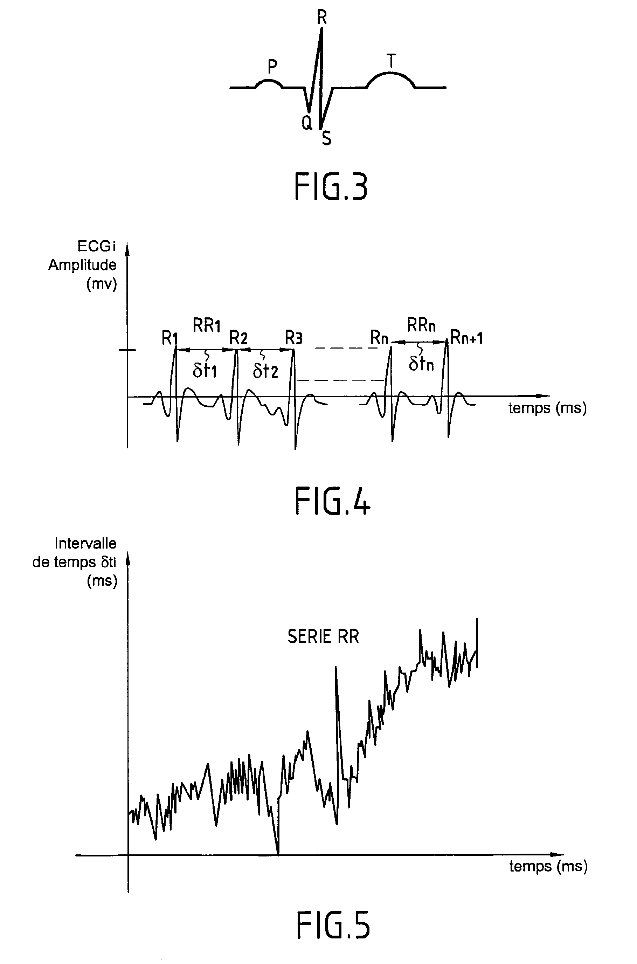

Un signal ECG est de manière connue constitué d'une succession de dépolarisations électriques dont l'allure est représentée sur la figure 3 annexée. L'onde P, qui correspond à la dépolarisation des oreillettes, présente une faible amplitude, et une forme de dôme. L'espace PQ traduit le temps de conduction auriculo-ventriculaire. Le complexe QRS reflète la contraction ventriculaire, et l'onde T la repolarisation ventriculaire. En pratique, on considère le pic R comme marqueur de la systole ventriculaire, c'est-à-dire du «battement cardiaque ».

En pratique, l'onde R étant le plus souvent la partie la plus fine et la plus ample du QRS, elle est généralement utilisée pour localiser ponctuellement le battement cardiaque avec une très bonne précision, en pratique de l'ordre du millième de seconde. Ainsi l'intervalle de temps entre deux ondes R successives caractérise de manière précise le temps séparant deux battements cardiaques successifs ; c'est la période du signal ECG, et l'inverse de cette période donne la fréquence cardiaque instantanée.

Pour construire automatiquement le signal, dit par la suite «série RR », représentant l'évolution dans le temps du rythme cardiaque instantané, on échantillonne le signal ECG qui est un signal analogique (conversion analogique/numérique du signal ECG), et on traite le signal ECG numérique échantillonné, en détectant automatiquement les ondes R dans ce signal numérique. Une série RR est ainsi de manière usuelle, constituée d'une pluralité d'échantillons (ou points) $(RR_i)$ successifs, chaque échantillon $(RR_i)$ correspondant à l'intervalle de temps, séparant deux ondes R successives du signal ECG.

Il faut toutefois souligner d'une part que l'on peut également utiliser les autres ondes de dépolarisation (P,Q, S ou T) du signal ECG pour caractériser la fréquence cardiaque, même si la précision de la mesure est moins bonne qu'en utilisant les ondes R.

D'autre part, en fonction de la technique d'acquisition choisie, le signal cardiaque peut présenter une forme différente de celle précitée d'un signal ECG. En conséquence, dans le présent texte, le terme « série RR » ne se limite pas à la définition particulière précitée basée sur les ondes R d'un signal ECG, mais se définit d'une manière plus générale dans le cadre de la présente invention comme une série de plusieurs échantillons dits $(RR_i)$, obtenue après échantillonnage d'un signal cardiaque analogique qui est caractéristique du rythme cardiaque, chaque échantillon $(RR_i)$ caractérisant l'intervalle de temps entre deux battements cardiaques successifs. Ainsi, une série RR, dans le cadre de l'invention peut être construite indifféremment à partir de tout type de signal cardiaque connu: signal ECG, signal cardiaque mesuré par capteur de pression sanglante ou par capteur de pouls infrarouge, signal cardiaque foetal mesuré par capteur ultrasonore ou par électrode au scalp, etc...

Analyse spectrale

L'analyse spectrale d'une série RR issue d'un signal cardiaque est de manière usuelle réalisée en deux étapes principales.

Dans une première étape, on calcule la courbe de densité spectrale de la série RR, par exemple entre 0 et 2Hz, en utilisant différentes méthodes connues. La méthode la plus couramment utilisée consiste à calculer la transformée de Fourier discrète rapide de la série RR, dans des fenêtres temporelles prédéfinies, pondérées au moyen d'une fenêtre de pondération prédéfinie. Il peut s'agir selon la réalisation envisagée d'une fenêtre de pondération rectangulaire, ou encore par exemple d'une fenêtre de pondération de Kaiser, Hamming ou Bartlett. Egalement, les fenêtres temporelles de calcul peuvent être prédéfinies et fixes, ou il peut s'agir d'une fenêtre temporelle de calcul, de taille prédéterminée, que l'on fait glisser dans le temps. Par exemple, la transformée de Fourrier est effectuée dans une fenêtre temporelle glissante de 256 secondes, appliquée sur la série RR, et soumise à une pondération de Kaiser pour limiter les effets de bord dus au fenêtrage.

Dans une seconde étape, à partir de la courbe de densité spectrale obtenue à l'issue de la première étape, on calcule automatiquement les puissance spectrales (aires sous la courbe de densité spectrale) entre des bornes de fréquences prédéterminées, et éventuellement réglables par un utilisateur.

Ces calculs de puissance spectrale permettent d'obtenir des informations quantitatives, qui sont caractéristiques de l'activité du Système Nerveux Autonome (SNA), et constituent ainsi un moyen d'investigation et d'analyse de la régulation cardiaque par le SNA. Par exemple on calcule une puissance spectrale basse fréquence (PS-BF) sur une plage de fréquences comprise entre 0,039 HZ et 0,148Hz, et une puissance spectrale haute fréquence (PS-HF) sur une plage de fréquences comprise entre 0,148HZ et 0,4Hz. On considère généralement que pour un adulte, la puissance spectrale basse fréquence (PS-BF) fournit une information quantitative caractéristique du tonus sympathique et parasympathique, tandis que la puissance spectrale haute fréquence (PS-HF) fournit une information quantitative caractéristique du tonus parasympathique.

La méthode d'analyse spectrale précitée présente plusieurs inconvénients.

Le calcul de la courbe de densité spectrale par transformée de Fourrier rapide (ou équivalent), est relativement lourd en termes de puissance de calcul et/ou temps de calcul, ce qui rend à ce jour cette méthode d'analyse spectrale inadaptée pour réaliser un système portatif miniaturisé et/ou difficile à mettre en oeuvre en temps réel.

Egalement, pour obtenir une résolution fréquentielle acceptable, la transformée de Fourier rapide doit être calculée dans des fenêtres temporelles relativement larges (par exemple 256s), ce qui correspond à un nombre important d'échantillons de la série RR. Il en découle que cette méthode d'analyse spectrale s'accompagne d'un effet « mémoire » qui retarde la prise en compte d'un changement intervenu dans le signal cardiaque.

La présente invention, qui est définie dans les revendication 1 à 12, a ainsi pour principal objectif de proposer une nouvelle méthode de traitement automatique d'une série RR, qui de manière comparable à la méthode d'analyse spectrale précitée, permet de calculer automatiquement à partir de la série RR au moins une information quantitative (paramètre) caractéristique de l'activité du SNA, mais qui pallie les inconvénients ci-dessus.

Cet objectif est atteint par une nouvelle méthode de traitement fréquentiel d'une série RR, selon laquelle on filtre la série RR, au moyen d'au moins un filtre numérique passe-bande ($F_k$) présentant une bande passante [fc ; f'c] prédéfinie, et on calcule un indice de variabilité ($I_k$) qui est fonction de l'amplitude instantanée [vs(n)] du signal discret ($S_k$) issu dudit filtre passe-bande.

Comparativement aux méthodes connues d'analyse spectrale, la méthode de l'invention présente l'avantage d'être plus simple à implémenter et nécessite moins de temps et/ou de puissance de calcul, tout en étant plus fine et en procurant un meilleur temps de réponse.

Dans une variante préférée de réalisation, l'indice de variabilité ($I_k$) est fonction de, et est de préférence égal à, la valeur efficace du signal discret ($S_k$) issu du filtre passe-bande.

Plus particulièrement selon l'invention, mais de manière non limitative de l'invention, on utilise un filtre numérique dont la bande passante est comprise dans l'une des bandes de fréquences suivantes :

- [0,04Hz ; 0,15Hz]
- [0,15Hz ; 2Hz] et de préférence [0,15Hz ; 0,5Hz]
- [0,05Hz ; 0,07Hz]
- [0,04Hz ; 2Hz], et de préférence [0,04Hz ; 0,5Hz]
- [0,03 Hz ; 0,15Hz], et de préférence [0,031Hz ; 0,102Hz]
- [0,1Hz ;2Hz], et de préférence [0,102Hz ;0,5Hz].

Dans une variante particulière de l'invention, le filtrage de la série RR est réalisé au moyen de plusieurs filtres ($F_1$, $F_2$,....), et on calcule un indice de variabilité fréquentielle qui est fonction de plusieurs indices de variabilité ($I_1$, $I_2$, ...) calculés en parallèle.

Plus particulièrement, dans une première variante de réalisation, le filtrage de la série RR est réalisé au moyen au moyen d'au moins deux filtres ($F_1$, $F_2$) dont les bandes passantes sont comprises respectivement dans les bandes de fréquences ci-après :

- $F_1$/[0,04Hz ; 0,15Hz]
- $F_2$ / [0,15Hz ; 2Hz] et de préférence [0,15Hz ; 0,5Hz],

et on calcule un indice de variabilité fréquentielle en fonction des indices de variabilité $I_1$ et $I_2$.

Dans une deuxième variante de réalisation, le filtrage de la série RR est réalisé au moyen d'au moins deux filtres ($F_1$, $F_2$) dont les bandes passantes sont comprises respectivement dans les bandes de fréquences ci-après :

- $F_1$/ [0,03 Hz ; 0,15Hz], et de préférence [0,031Hz ; 0,102Hz]
- $F_2$/ [0,1Hz ;2Hz], et de préférence [0,102Hz ;0,5Hz],

Et on calcule un indice de variabilité fréquentielle en fonction des indices de variabilité $I_1$ et $I_2$.

L'invention a pour autre objet un procédé d'acquisition et de traitement électronique d'un signal cardiaque analogique caractéristique du rythme cardiaque. Ce procédé est connu en ce qu'on enregistre le signal cardiaque, on numérise ce signal, et on construit une série RR.

De manière caractéristique selon l'invention, on traite automatiquement la série RR en temps réel, au fur et à mesure de la construction de cette série, en mettant en oeuvre la méthode de traitement fréquentiel précitée de l'invention.

L'invention a enfin pour objet un système d'acquisition et de traitement en temps réel d'un signal cardiaque, lequel système comporte des moyens d'acquisition permettant l'acquisition d'un signal cardiaque, des premiers moyens électroniques permettant de construire automatiquement, une série RR constituée d'une pluralité d'échantillons ($RR_i$) définissant respectivement les intervalles de temps qui séparent deux battements cardiaques successifs.

De manière caractéristique selon l'invention, le système d'acquisition et de traitement comprend en outre des deuxièmes moyens électroniques conçus pour traiter la série RR délivrée par les premiers moyens électroniques, conformément à la méthode de traitement fréquentiel de l'invention.

Selon une application préférentielle de l'invention, le système d'acquisition et de traitement constitue un cardiotocographe intégrant une fonction nouvelle de mesure de la souffrance foetale.

D'autres caractéristiques et avantages de l'invention apparaîtront plus clairement à la lecture de la description ci-après de deux variantes préférées de réalisation, laquelle description est faite en référence aux dessins annexés sur lesquels :

- la figure 1 représente de manière schématique les principaux éléments d'un système d'acquisition et de traitement fréquentiel d'un signal ECG conforme à l'invention,
- la figure 2 est un synoptique des trois principaux modules fonctionnels du logiciel de traitement exécuté par l'unité de traitement du système d'acquisition de la figure 1 ,

- la figure 3 représente l'onde (PQRST) caractéristique d'un signal ECG analogique,
- la figure 4 représente un exemple de signal numérique $ECG_i$ obtenu après échantillonnage d'un signal ECG analogique,
- la figure 5 représente la série RR construite à partir du signal de la figure 4, et
- la figure 6 est un synoptique d'un cardiotocographe qui a été perfectionné en sorte de remplir une fonction supplémentaire de suivi en temps réel de la souffrance foetale.

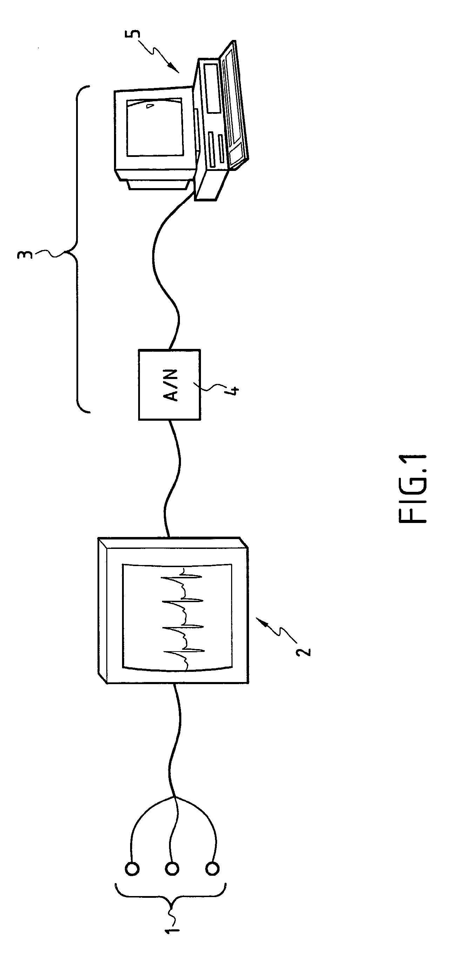

On a représenté sur la figure 1, un système d'acquisition et de traitement fréquentiel du rythme cardiaque. Ce système comporte :

- des moyens usuels d'acquisition d'un signal ECG, comprenant plusieurs électrodes de mesure 1 reliées en entrée à un moniteur électrocardiographique (ECG) 2,
- des moyens 3 de traitement en temps réel du signal ECG délivré en sortie par le moniteur ECG 2.

Les moyens de traitement 3 du signal ECG comprennent un convertisseur analogique/numérique 4, et une unité de traitement programmée 5. L'entrée du convertisseur 4 est reliée à la sortie du moniteur ECG 2, et la sortie du convertisseur 4 est reliée à un port d'entrée de l'unité de traitement 5. Dans un exemple particulier de réalisation, non limitatif de l'invention, l'unité de traitement 5 est constituée par un micro-ordinateur, le convertisseur 4 étant relié à un port série RS232 de ce micro-ordinateur.

En fonctionnement, les électrodes 1 sont appliquées sur le corps d'un patient, et le moniteur ECG délivre en sortie de manière usuelle un signal électrique analogique, dit signal ECG, qui pour chaque battement cardiaque, à la forme du signal représenté à la figure 3. Ce signal ECG est numérisé par le convertisseur 4, avec une fréquence d'échantillonnage (f) prédéterminée, valant par exemple 256 Hz. Ce signal numérique (représenté sur la figure 4) est ensuite traité en temps réel par l'unité de traitement 5, au moyen d'un logiciel de traitement spécifique décrit en détail ultérieurement.

Signal analogique ECG / Figure 3

Le signal électrocardiographique (ECG), délivré en sortie par le moniteur électrocardiographique 2, est constitué d'un ensemble d'ondes électriques dont l'allure est donnée sur la figure 3.

L'onde P qui correspond à la dépolarisation des oreillettes, a une faible amplitude et une forme de dôme ; l'espace PQ qui traduit le temps de conduction auriculo-ventriculaire ; le complexe QRS reflète la contraction ventriculaire, et l'onde T la repolarisation ventriculaire. En pratique on considère l'onde R comme marqueur de la systole ventriculaire, ou du «battement cardiaque ».

L'intervalle RR/ figure 4

L'intervalle « RR » correspond au temps séparant deux battements cardiaques, c'est la période instantanée du signal, et son inverse donne la fréquence cardiaque instantanée. L'onde R, étant le plus souvent la partie la plus fine et la plus ample du QRS, permet de localiser ponctuellement le battement cardiaque avec une très bonne précision (de l'ordre du millième de seconde).

L'enregistrement de la succession des ondes R, à partir du signal ECG, permet de construire la série RR et de l'analyser dans le domaine fréquentiel.

Synoptique général du logiciel de traitement du signal ECG numérisé

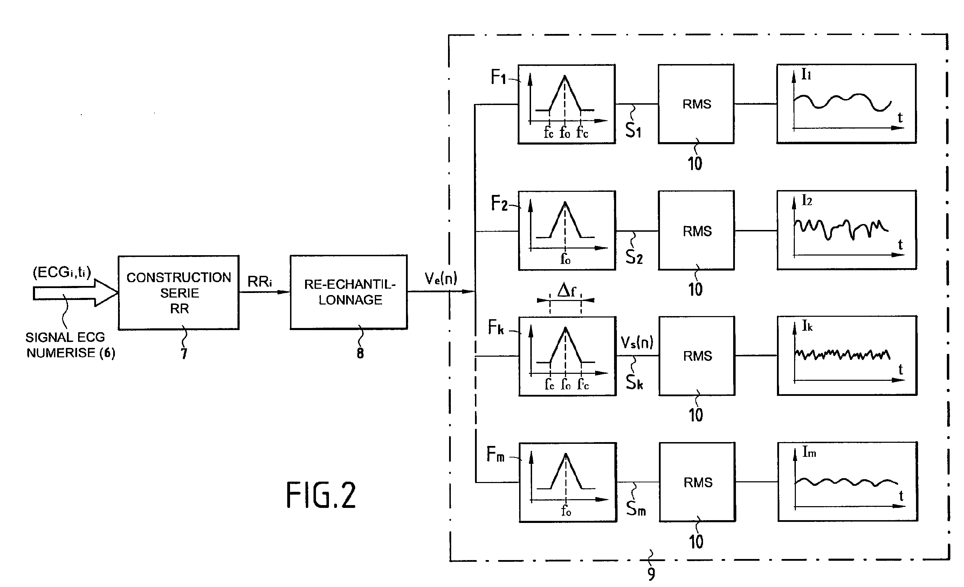

On a représenté sur la figure 2, les trois principaux modules fonctionnels 7, 8, 9 du logiciel de traitement du signal ECG numérisé.

Le premier module 7 est alimenté en entrée et en temps réel avec les données numériques successives constitutives du signal ECG numérisé 6, et délivrées par le convertisseur analogique numérique 4. Ces données sont formées, pour chaque échantillon résultant de la conversion numérique du signal analogique ECG, de l'amplitude instantanée $ECG_i$ du signal ECG, et de l'instant $t_i$ d'échantillonnage ($t_i = n_i/f$, avec $n_i$ numéro d'échantillon et f représentant la fréquence d'échantillonnage du convertisseur 4).

Le premier module 7 est conçu pour détecter automatiquement chaque pic $R_i$ successif dans le signal numérique 6, et pour construire automatiquement une série RR à partir de ce signal. En sortie, ce module 7 délivre successivement dans le temps, les points $RR_i$ successifs de la série RR. La valeur de chaque point $RR_i$ est égale à l'intervalle de temps ($\delta t_i$) (exprimé en multiple de la fréquence d'échantillonnage f) séparant un pic $R_i$ du pic suivant $R_{i+1}$ (dans une autre variante il pourrait s'agir du pic précédent $R_{i-1}$).

Le deuxième module 8 a pour fonction de ré-échantilloner la série RR issue du module 7 à une fréquence prédéfinie (par exemple 4hz, ce qui correspond à une période d'échantillonnage Ts de 250ms), en sorte d'obtenir en sortie une série RR dont les échantillons $RR_i$ sont équidistants d'un point de vue temporel, c'est-à-dire en d'autres termes une série RR dont les instants d'échantillonnages sont réguliers. Ce module 8 (connu en soi) réalise ce ré-échantillonnage par interpolation linéaire.

Sachant que le module 7 réalisant la fonction précitée de détection des pics $R_i$ et de construction de la série RR et le module 8 de ré-échantillonnage de la série RR sont par ailleurs déjà connus, et mis en oeuvre notamment dans les solutions d'analyse spectrale de l'art antérieur qui reposent sur un calcul de transformée de Fourier discrète, ces deux modules 7 et 8 ne seront pas plus amplement décrits dans le présent texte.

Le troisième module 9 est un module de traitement fréquentiel de la série RR ré-échantillonnée, qui implémente la méthode de traitement fréquentiel de l'invention.

Ce module 9 comporte en parallèle (m) filtres numériques passe-bande $F_1$ à $F_m$, chaque filtre étant caractérisé par une bande passante prédéfinie [fc ;f'c] qui lui est propre (fc et f'c représentent les fréquences de coupure du filtre) . En sortie de chaque filtre $F_1$ à $F_m$, on obtient un signal discret filtré ($S_1$ à $S_m$) comportant uniquement des composantes fréquentielles dans la bande passante du filtre.

Dans le cadre de l'invention, tout type connu de filtre numérique passe-bande peut être utilisé. On peut utiliser un filtre numérique passe-bande composé par exemple d'un filtre passe haut (fréquence de coupure fc) et d'un filtre passe-bas (fréquence de coupure f'c) en série.

Dans une variante préférée de réalisation chaque filtre ($F_1$ à $F_m$) est un filtre sélectif récursif à réponse impulsionnelle infinie (RII), présentant une bande passante étroite de largeur $\Delta f$, et centrée sur une fréquence de filtrage centrale $f_0$.

Plus particulièrement, mais de manière non limitative, dans un exemple particulier de réalisation, la fonction de transfert H(w) de chaque filtre est définie par l'équation suivante :

$$H(w) = \frac{1}{1 + j.Q.(\frac{w}{w_0} - \frac{w_0}{w})}$$

dans laquelle

$w = 2\pi f$, avec f représentant la fréquence du signal ;

$w_0 = 2\pi f_0$, avec $f_0$ représentant la fréquence centrale de la bande passante ;

$Q = \frac{f_0}{\Delta f}$ avec $\Delta f$ représentant la largeur de la bande passante du filtre à -3dB.

En appliquant la transformation bilinéaire sur H(w) on obtient l'équation récurrente ci-après du filtre numérique qui donne, pour chaque période d'échantillonnage n, l'échantillon de sortie vs(n) en fonction des échantillons d'entrée ve(n) et ve(n-2) et des échantillons de sortie vs(n-1) et vs (n-2) :

$$vs(n) = A.ve(n) + B.ve(n-2) + C.vs(n-1) + D.vs(n-2),$$

Avec

$$A = \frac{1}{d_0} \; ;$$

B = - A ;

$$C = \frac{\dfrac{4.Q}{w_0.Ts} - w_0.Q.Ts}{d_0} \quad ;$$

$$D = \frac{1 - \dfrac{2.Q}{w_0.Ts} - \dfrac{w_0.Q.Ts}{2}}{d_0} \quad ;$$

$$d_0 = 1 + \frac{2.Q}{w_0.Ts} + \frac{w_0.Q.Ts}{2} \quad ;$$

Ts représentant la période de ré-échantillonnage de la série RR.

Chaque signal discret filtré ($S_1$ à $S_m$) est ensuite traité par un module numérique 10, qui est conçu pour calculer un indice de variabilité ($I_1$ à $I_m$).

Dans l'exemple particulier illustré sur la figure 2, et donné à titre d'exemple préféré de réalisation chaque indice de variabilité ($I_1$ à $I_m$) correspond à la valeur efficace du signal discret filtré correspondant ($S_1$ à $S_m$). Pour réaliser ce calcul de la valeur efficace, chaque module 10 est par exemple programmé (algorithme rms) pour calculer pour chaque signal discret filtre $S_k$, un indice de variabilité $I_k$ selon la formule suivante, calculée dans une fenêtre de calcul glissante d'une période $T_0$ :

$$I_k = \sqrt{\frac{1}{N} \sum_{i=1}^{N} Vs^2(i)}$$

avec :

   N représentant le nombre d'échantillons sur un période $T_0 = 1/f_0$,
   Vs(i) étant la valeur numérique de chaque échantillon de sortie du filtre (signal $S_k$).

Il a été vérifié que l'indice de variabilité $I_k$, calculé sur la base de l'algorithme rms précité, à partir d'un signal ($S_k$) issu d'un filtre passe-bande ($F_k$) était sensiblement égal à l'indice de variabilité usuellement calculé dans les solutions de l'art antérieur, qui sont basées sur un calcul de la courbe de densité spectrale par transformée de Fourier rapide, suivie d'un calcul de puissance spectrale (aire sous la courbe de densité spectrale) dans la bande de fréquences[fc,f'c].

La solution de l'invention peut ainsi avantageusement être substituée aux solutions de l'art antérieur pour l'étude et/ou le suivi du rythme cardiaque, et présente l'avantage d'être plus fine et d'offrir un meilleur temps de réponse (suppression de l'effet mémoire inhérent aux méthodes basées sur un calcul de transformée de Fourier ou équivalent). La solution de l'invention est également plus simple à implémenter et plus rapide en temps de calcul. Les algorithmes de filtrage numérique et les algorithmes de calcul de l'indice de variabilité utilisés dans la méthode de l'invention d'une part peuvent plus facilement être prévus pour fonctionner en temps réel, et d'autre part peuvent facilement être implémentés dans un système de surveillance du rythme cardiaque de petite taille, et notamment de type ambulatoire.

A titre de variantes préférées de réalisation vont à présent être donnés différents exemples particuliers de filtres sélectifs récursif RII ($F_k$), qui sont particulièrement intéressants pour l'étude du rythme cardiaque.

Filtre $F_1$: fc= 0,04Hz ;f'c= 0,15Hz ; $f_0$ = 0,095Hz ; $\Delta f$ = 0,11Hz
Filtre $F_2$: fc= 0,15Hz ;f'c= 0,5Hz ; $f_0$ = 0,325Hz ; $\Delta f$ = 0,35Hz
Filtre $F_3$ : fc=0,04Hz ; f'c= 0,5 Hz ; $f_0$= 0,27 Hz $\Delta f$ = 0,46Hz.

L'indice de variabilité $I_1$ calculé sur la base de l'algorithme rms précité, à partir du signal issu du filtre particulier

précité $F_1$, est un paramètre quantitatif qui permet de caractériser, chez un adulte, l'évolution dans le temps du tonus sympathique et parasympathique.

L'indice de variabilité $I_2$ calculé sur la base de l'algorithme rms précité, à partir du signal issu du filtre particulier précité $F_2$, est un paramètre quantitatif qui permet de caractériser, chez un adulte, l'évolution dans le temps du tonus parasympathique.

L'indice de variabilité $I_3$ calculé sur la base de l'algorithme rms précité, à partir respectivement de signaux issu du filtre particulier précité $F_3$, est un paramètre quantitatif qui donne une indication sur la variation de temps entre deux battements cardiaques successifs (variation battement à battement).

Bien que dans le cadre de l'invention, le calcul d'un indice de variabilité $I_k$ basé sur un calcul de valeur efficace donne des résultats particulièrement intéressant d'un point du vue de la quantification de l'activité cardiaque, il convient néanmoins de souligner que l'invention n'est toutefois pas limitée au calcul d'un indice de variabilité ($I_k$) basé sur la valeur efficace de chaque signal discret filtré, mais s'étend à tout calcul d'un indice de variabilité ($I_k$) qui est fonction de l'amplitude instantanée vs(n) du signal discret filtré correspondant ($S_k$). Egalement, d'autres plages de fréquences pour la bande passante d'un filtre peuvent s'avérer intéressantes, l'invention n'étant pas limitée aux plages de fréquences particulières précitées.

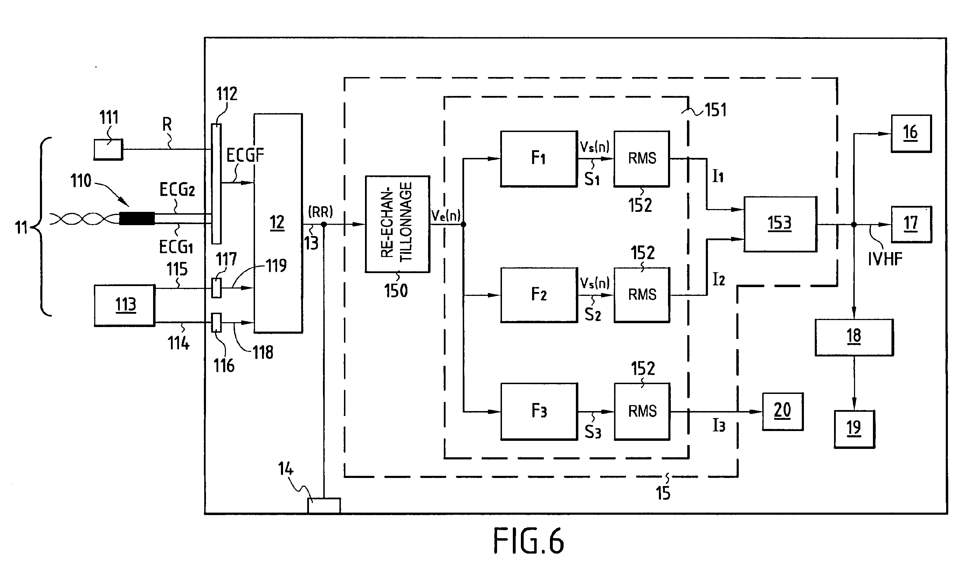

On a représenté sur la figure 6, une autre application de l'invention dans le domaine de la surveillance de la souffrance foetale. Sur cette figure 6 est représenté de manière schématique le synoptique d'un cardiotocographe qui permet de manière usuelle de mesurer le signal cardiaque d'un foetus, et qui de manière nouvelle selon l'invention a été perfectionné en sorte de remplir une fonction supplémentaire de suivi en temps réel de la souffrance foetale.

Le cardiotocographe comporte de manière usuelle :

- des moyens 11 d'acquisition du signal cardiaque foetal ;
- une unité de traitement 12 (sous la forme par exemple d'un microprocesseur ou microcontrôleur) qui reçoit en entrée les signaux de mesure délivrés par les moyens d'acquisition 11, et qui traite de manière usuelle lesdits signaux de mesure et délivre en sortie un signal 13, de type numérique, formant une série RR ;

Les moyens 11 d'acquisition du signal cardiaque foetal sont usuels et comportent :

- une électrode double spire 110 qui, en fonctionnement, est fixée, par voie vaginale sur le scalp foetal, et qui délivre deux signaux de mesure analogiques ECG1 et ECG2,
- une électrode de référence 111 qui, en fonctionnement, est placée au contact de la cuisse maternelle, et qui délivre un signal de mesure R, représentatif de la pulsation cardiaque maternelle et servant de signal de référence,
- des moyens de traitement électronique 112, qui délivrent en sortie, à partir des signaux précités ECG1, ECG2 et R, un signal numérique

ECGF représentant le signal électrocardiographique foetal numérisé. Les moyens 11 d'acquisition comportent également :

- une sangle abdominale 113 qui, en fonctionnement, est placée sur l'abdomen maternel, et qui intègre deux capteurs : un capteur ultrasonore permettant une mesure par ultrasons du rythme cardiaque foetal (signal de mesure analogique 114), et un capteur de pression qui permet de mesurer les contraction utérines (signal de mesure analogique 115),
- des circuits électroniques 116,117 usuels permettant de traiter (notamment amplification et échantillonnage) les signaux analogiques précités 114 et 115, et délivrant respectivement des signaux de mesure numérisés 118,119.

L'unité de traitement 12 du cardiotocographe est programmée notamment pour construire, à partir soit du signal électrocardiographique foetal numérisé ECGF, soit du signal de mesure 118, une série RR (signal numérique 13) qui est constituée d'une succession d'échantillons ($RR_i$) définissant respectivement les intervalles de temps qui séparent deux battements cardiaques foetals successifs. Ce signal 13 (série RR) est de manière usuelle disponible sur un port de sortie 14 (port série ou parallèle) du cardiotocographe.

Le cardiotocographe de la figure 6 est nouveau en ce qu'il intègre un module fonctionnel supplémentaire 15 qui permet de traiter en temps réel le signal 13 (série RR) délivré par l'unité de traitement 12.

Ce module 15 comporte un premier sous-module 150 de ré-échantillonnage de la série RR, qui est identique au module 8 précité de la variante de la figure 2, et un deuxième sous-module 151 de traitement fréquentiel de la série RR, qui est comparable au module 9 précédemment décrit de la variante de la figure 2.

Plus particulièrement ce deuxième sous-module 151 comporte trois filtres numériques sélectifs récursifs RII ($F_1$, $F_2$,$F_3$) en parallèle.

Dans une première variante de réalisation :

Filtre $F_1$ : fc= 0,04Hz ; f'c= 0,15Hz ; $f_0$ = 0,095Hz $\Delta f$ = 0,11Hz.

Filtre $F_2$ : fc= 0,15Hz ; f'c= 0,5Hz ; $f_0$ = 0,325Hz $\Delta f$ = 0,35Hz

Filtre $F_3$ : fc= 0,05Hz ; f'c= 0,066Hz ; $f_0$ = 0,058Hz ; $\Delta f$ = 0,116Hz.

Dans une deuxième variante de réalisation :

Filtre $F_1$ : fc= 0,031Hz ; f'c= 0,102Hz ; $f_0$ = 0,0665Hz $\Delta f$ = 0,071Hz.

Filtre $F_2$ ; c= 0,102Hz ; f'c= 0,5Hz ; $f_0$ = 0,301Hz $\Delta f$ = 0,398Hz

Filtre $F_3$ : fc= 0,05Hz ; f'c= 0,066Hz ; $f_0$ = 0,058Hz ; $\Delta f$ = 0,016Hz

Les trois signaux discrets $S_1$, $S_2$ et $S_3$ issus des filtres $F_1$, $F_2$ et $F_3$ sont traités en parallèle par trois modules 152 de calcul de la valeur efficace de ces signaux (conformément à l'algorithme rms précédemment décrit pour la variante de la figure 2).

Le module 15 comporte enfin un module 153, qui permet de calculer, à partir des indice de variabilité I1 et I2 calculés en parallèle par les modules 152, un indice de variabilité haute fréquence (IVHF) caractéristique de la souffrance foetale.

Le calcul de l'indice IVHF est de préférence basé sur l'une ou l'autre des équations (1), (2) ci-après :

$$(1) \quad IVHF = \frac{I_2}{I_1}$$

$$(2) \quad IVHF = \frac{I_2}{I_1 + I_2}$$

En référence à la figure 6, l'indice IVHF est fourni en temps réel à un utilisateur du cardiotocographe, de deux manières différentes et non exhaustives :

- la valeur instantanée de l'indice IVHF est affichée en façade du cardiotocographe sur un afficheur classique 16 ;
- l'évolution dans le temps de l'indice IFVH est imprimée sous forme d'une courbe temporelle sur une bande de papier, au moyen d'une imprimante 17.

L'indice IVHF permet de quantifier la souffrance foetale ; En deçà d'un seuil prédéterminé, on peut montrer que le foetus est dans une phase de souffrance foetale. Ainsi, le praticien qui utilise le cardiotocographe est informé en temps réel de la valeur de cet indice IVHF, et par là même du niveau de souffrance foetal.

Egalement dans la variante de réalisation de la figure 6, le cardiotocographe comporte des moyens 18 de détection de l'indice IVHF délivré par les moyens de calcul 153, lesquels moyens de détection sont conçus pour comparer la valeur instantanée de l'indice IVHF avec au moins un seuil prédéterminé, de préférence réglable par l'utilisateur du cardiotocographe. Lorsque la valeur de l'indice IVHF est inférieure à ce seuil prédéterminé, les moyens de détection 18 pilotent des moyens d'alarme, qui peuvent être de type visuel et/ou sonore, et qui permettent d'alerter le praticien.

En parallèle au calcul et au traitement de l'indice IVHF précité, l'indice de variabilité $I_3$ qui est à partir du signal $S_3$ issu du filtre $F_3$ précité est un paramètre quantitatif qui permet également de caractériser la souffrance foetale. Ce paramètre est néanmoins moins précis que l'indice IVFH précité. La valeur instantanée de cet indice $I_3$ est affiché pour le praticien sur afficheur classique 20.

Le module 15 qui permet le calcul des indices IVHF et $I_3$ à partir de la série RR peut être un module électronique rapporté que l'on vient connecter sur la carte électronique mère comportant l'unité de traitement 12 ; l'architecture électronique de ce module 15 peut être basée sur l'utilisation d'un microcontrôleur ou d'un microprocesseur ; il peut encore s'agir d'un circuit programmable de type EPLD ou FPGA, ou encore d'un circuit intégré spécifique (ASIC) spécialement développé pour remplir les fonctions précédemment décrites du module 15. Egalement, les fonctions du module 15 pourraient être réalisées par l'unité de traitement 12, le fonctionnement de cette unité de traitement 12 étant modifié par exemple par l'ajout d'une mémoire lui permettant d'assurer les nouvelle fonctionnalités du module 15. Enfin, dans une autre variante de réalisation, le module 15 pourrait être un périphérique externe au cardiotocographe, et raccordé à l'unité de traitement 12 dudit cardiotocographe par le port de communication 14.

**Revendications**

1. Méthode de traitement fréquentiel d'une série RR dans laquelle on filtre la série RR, au moyen d'au moins un filtre numérique passe-bande ($F_k$) présentant une bande passante [fc ; f'c] prédéfinie, **caractérisée en ce qu'**on calcule un indice de variabilité ($I_k$) qui est fonction de l'amplitude instantanée [vs(n)] du signal discret ($S_k$) issu dudit filtre passe-bande et qui est fonction de, et est de préférence égal à, la valeur efficace du signal discret ($S_k$) issu du filtre passe-bande.

2. Méthode selon la revendication 1 **caractérisée en ce qu'**on utilise un filtre sélectif récursif à réponse impulsionnelle infinie.

3. Méthode selon l'une des revendications 1 à 2 **caractérisée en ce qu'**on utilise un filtre numérique dont la bande passante est comprise dans la bande de fréquences [0 ; 2Hz].

4. Méthode selon la revendication 3 **caractérisée en ce qu'**on utilise un filtre numérique dont la bande passante est comprise dans l'une des bandes de fréquences suivantes :

   - [0,04Hz ; 0,15Hz]
   - [0,15Hz ; 2Hz] et de préférence [0,15Hz ; 0,5Hz]
   - [0,05Hz ; 0,07Hz]
   - [0,04Hz ; 2Hz ], et de préférence [0,04Hz ; 0,5Hz]
   - [0,03 Hz ; 0,15Hz], et de préférence [0,031Hz ; 0,102Hz]
   - [0,1Hz ;2Hz], et de préférence [0,102Hz ;0,5Hz].

5. Méthode selon l'une des revendications 1 à 4 **caractérisée en ce que** le filtrage de la série RR est réalisé au moyen de plusieurs filtres ($F_1$, $F_2$,.... ), et **en ce qu'**on calcule un indice de variabilité fréquentielle qui est fonction de plusieurs indices de variabilité ($I_1$, $I_2$, ...) calculés en parallèle.

6. Méthode selon la revendication 5 **caractérisée en ce que** le filtrage de la série RR est réalisé au moyen d'au moins deux filtres ($F_1$, $F_2$) dont les bandes passantes sont comprises respectivement dans les bandes de fréquences ci-après :

   - $F_1$/[0,04Hz ; 0,15Hz]
   - $F_2$ /[0,15Hz ; 2Hz] et de préférence [0,15Hz ; 0,5Hz],

   et **en ce qu'**on calcule un indice de variabilité fréquentielle en fonction des indices de variabilité $I_1$ et $I_2$.

7. Méthode selon la revendication 5 **caractérisée en ce que** le filtrage de la série RR est réalisé au moyen d'au moins deux filtres ($F_1$, $F_2$) dont les bandes passantes sont comprises respectivement dans les bandes de fréquences ci-après :

   - $F_1$/ [0,03 Hz ; 0,15Hz], et de préférence [0,031Hz ; 0,102Hz]
   - $F_2$/ [0,1Hz ;2Hz], et de préférence [0,102Hz ;0,5Hz],

   et **en ce qu'**on calcule un indice de variabilité fréquentielle en fonction des indices de variabilité $I_1$ et $I_2$.

8. Procédé d'acquisition et de traitement électronique d'un signal cardiaque analogique caractéristique du rythme cardiaque, selon lequel on enregistre le signal cardiaque, on numérise ce signal et on construit une série RR, **caractérisé en ce qu'**on traite automatiquement la série RR en temps réel, au fur et à mesure de la construction de cette série, en mettant en oeuvre la méthode de traitement fréquentiel visée à l'une quelconque des revendications 1 à 7.

9. Procédé selon la revendication 8 **caractérisé en ce que** le signal cardiaque analogique est un signal cardiaque foetal.

10. Système d'acquisition et de traitement en temps réel d'un signal cardiaque, lequel système comporte des moyens d'acquisition (1 / 11) permettant l'acquisition d'un signal cardiaque, des premiers moyens électroniques (2,4/12) permettant de construire automatiquement, une série RR constituée d'une pluralité d'échantillons ($RR_i$) définissant respectivement les intervalles de temps qui séparent deux battements cardiaques successifs, **caractérisé en ce qu'**il comprend en outre des deuxièmes moyens électroniques (5/15) conçus pour traiter la série RR délivrée par les premiers moyens électroniques conformément à la méthode de traitement fréquentiel visée à l'une quelconque des revendications 1 à 7.

11. Système selon la revendication 10 **caractérisé en ce que** les moyens d'acquisition (11) sont conçus pour permettre l'acquisition du signal cardiaque d'un foetus, **en ce que** les deuxièmes moyens électroniques (15) sont conçus pour traiter la série RR délivrée par les premiers moyens électroniques conformément à la méthode de traitement fréquentiel visée à l'une des revendications à 5 à 7.

12. Système selon la revendication 11 **caractérisé en ce qu'**il constitue un cardiotocographe intégrant une fonction de mesure de la souffrance foetale.

**Patentansprüche**

1. Verfahren zur Frequenzverarbeitung einer RR-Serie, wobei die RR-Serie mittels zumindest eines digitalen Bandpassfilters ($F_k$) mit einem vorbestimmten Durchlassband [fc; f'c] gefiltert wird, **dadurch gekennzeichnet, dass** ein Variabilitätsindex ($I_k$) berechnet wird, der eine Funktion von der momentanen Amplitude [vs(n)] des diskreten Signals ($S_k$) oder des Momentanwertes davon ist, das vom Bandpassfilter abgegeben wird, und eine Funktion von, vorzugsweise gleich dem Effektivwert des diskreten Signals ($S_k$) von dem oder aus dem Bandpassfilter ist.

2. Verfahren nach Anspruch 1, **dadurch gekennzeichnet, dass** ein selektives rekursives Filter mit unendlicher Impulsantwort verwendet wird.

3. Verfahren nach einem der Ansprüche 1 bis 2, **dadurch gekennzeichnet, dass** ein digitales Filter verwendet wird, bei dem das Durchlassband im Frequenzband [0; 2 Hz] liegt.

4. Verfahren nach Anspruch 3, **dadurch gekennzeichnet, dass** ein digitales Filter verwendet wird, bei dem das Durchlassband in einem der nachfolgenden Frequenzbänder liegt:

   - [0,04 Hz; 0,15 Hz]
   - [0,15 Hz; 2 Hz], vorzugsweise [0,15 Hz; 0,5 Hz]
   - [0,05 Hz; 0,07 Hz]
   - [0,04 Hz; 2 Hz], vorzugsweise [0,04 Hz; 0,5 Hz]
   - [0,03 Hz; 0,15 Hz], vorzugsweise [0,031 Hz; 0,102 Hz]
   - [0,1 Hz; 2 Hz], vorzugsweise [0,102 Hz; 0,5 Hz].

5. Verfahren nach einem der Ansprüche 1 bis 4, **dadurch gekennzeichnet, dass** die Filterung der RR-Serie mittels mehrerer Filter ($F_1$, $F_2$, ...) erfolgt und dass ein Frequenzvariabilitätsindex berechnet wird, der Funktion von mehreren Variabilitätsindizes ($I_1$, $I_2$, ...) ist, die parallel berechnet werden.

6. Verfahren nach Anspruch 5, **dadurch gekennzeichnet, dass** die Filterung der RR-Serie mittels zweier Filter ($F_1$, $F_2$) erfolgt, bei denen die Durchlassbänder jeweils in dem jeweiligen nachfolgenden Frequenzband liegen:

   - F1/ [0,04 Hz; 0,15 Hz]
   - F2/ [0,15 Hz; 2 Hz], vorzugsweise [0,15 Hz; 0,5 Hz],

   und dass ein Frequenzvariabilitätsindex in Abhängigkeit von den Variabilitätsindizes $I_1$ und $I_2$ berechnet wird.

7. Verfahren nach Anspruch 5, **dadurch gekennzeichnet, dass** die Filterung der RR-Serie mittels zweier Filter ($F_1$,

$F_2$) erfolgt, bei denen das Durchlassband jeweils im nachfolgenden Frequenzband liegt:

  - F1/ [0,03 Hz; 0,15 Hz], vorzugsweise [0,031 Hz; 0,102 Hz]
  - F2/ [0,1 Hz; 2 Hz], vorzugsweise [0,102 Hz; 0,5 Hz],

und dass ein Frequenzvariabilitätsindex in Abhängigkeit oder als Funktion von den Variabilitätsindizes $I_1$ und $I_2$ berechnet wird.

8. **Verfahren** zur elektronischen Erfassung und Verarbeitung eines für den Herzrhythmus charakteristischen analogen Herzsignals, wobei das Herzsignal aufgezeichnet wird, dieses Signal digitalisiert wird und eine RR-Serie konstruiert wird, **dadurch gekennzeichnet, dass** die RR-Serie automatisch in Echtzeit nach und nach im Laufe oder nach Maßgabe der Konstruktion dieser Serie verarbeitet wird, indem das Verfahren der Frequenzverarbeitung nach einem der Ansprüche 1 bis 7 durchgeführt wird.

9. Verfahren nach Anspruch 8, **dadurch gekennzeichnet, dass** das analoge Herzsignal ein fetales Herzsignal ist.

10. **System** zur Erfassung und Verarbeitung eines Herzsignals in Echtzeit, wobei das System Erfassungsmittel (1 / 11) aufweist, mit denen die Erfassung eines Herzsignals möglich ist, sowie erste elektronische Mittel (2, 4/12), mit denen eine RR-Serie automatisch konstruiert werden kann, die aus einer Mehrzahl von Stichproben ($RR_l$) besteht, die jeweils die Zeitintervalle definieren, die zwei aufeinanderfolgende Herzschläge trennen, **dadurch gekennzeichnet, dass** es ferner zweite elektronische Mittel (5/15) aufweist, ausgebildet, von den ersten elektronischen Mitteln bereitgestellte RR-Serie gemäß dem Verfahren der Frequenzverarbeitung nach einem der Ansprüche 1 bis 7 zu verarbeiten.

11. System nach Anspruch 10, **dadurch gekennzeichnet, dass** die Erfassungsmittel (11) ausgebildet sind, die Erfassung des Herzsignals eines Fetus zu gestatten, dass die zweiten elektronischen Mittel (15) dazu ausgelegt sind, die von den ersten elektronischen Mitteln bereitgestellte RR-Serie gemäß dem Verfahren der Frequenzverarbeitung nach einem der Ansprüche 5 bis 7 zu verarbeiten.

12. System nach Anspruch 11, **dadurch gekennzeichnet, dass** es einen Kardiotokographen darstellt, der eine Funktion zum Messen von Fetal-Distress-Situationen (fetales oder fötales Leiden oder Notsituation) umfasst.

## Claims

1. A frequency processing method in an RR series in which the RR series is filtered, using at least one digital bandpass filter ($F_k$) having a predefined passband [fc; f'c], **characterized in that** a variability index ($I_k$) is calculated that depends on the instantaneous amplitude [vs(n)] of the discrete signal ($S_k$) from said bandpass filter, and which depends on, and is preferably equal to, the effective value of the discrete signal ($S_k$) from the bandpass filter.

2. The method according to claim 1, **characterized in that** a recursive selective filter with infinite impulse response is used.

3. The method according to one of claims 1 to 2, **characterized in that** a digital filter is used whereof the passband is comprised in the frequency range [0; 2 Hz].

4. The method according to claim 3, **characterized in that** a digital filter is used whereof the passband is comprised in one of the following frequency bands:

    - [0.04 Hz; 0.15 Hz]
    - [0.15 Hz; 2 Hz], and preferably [0.15 Hz; 0.5 Hz]
    - [0.05 Hz; 0.07 Hz]
    - [0.04 Hz; 2 Hz], and preferably [0.04 Hz; 0.5 Hz]
    - [0.03 Hz; 0.15 Hz], and preferably [0.031 Hz; 0.102 Hz]
    - [0.1 Hz; 2 Hz], and preferably [0.102 Hz; 0.5 Hz].

5. The method according to one of claims 1 to 4, **characterized in that** the filtering of the RR series is done using several filters ($F_1$, $F_2$,...), and **in that** a frequency variability index is calculated that depends on several variability

indices ($I_1$, $I_2$, ...) calculated in parallel.

6. The method according to claim 5, **characterized in that** the filtering of the RR series is done using at least two filters ($F_1$, $F_2$) whereof the passband is respectively comprised in the frequency bands below:

   - $F_1$/[0.04 Hz; 0.15 Hz]
   - $F_2$/[0.15 Hz; 2 Hz], and preferably [0.15 Hz; 0.5 Hz],

   and **in that** a frequency variability index is calculated as a function of the variability indices $I_1$ and $I_2$.

7. The method according to claim 5, **characterized in that** the filtering of the RR series is done using at least two filters ($F_1$, $F_2$) whereof the passbands are respectively comprised in the frequency bands below:

   - $F_1$/[0.03 Hz; 0.15 Hz], and preferably [0.031 Hz; 0.102 Hz]
   - $F_2$/[0.1 Hz; 2 Hz], and preferably [0.102 Hz; 0.5 Hz],

   and **in that** the frequency variability index is calculated as a function of the variability indices $I_1$ and $I_2$.

8. An acquisition and electronic processing method for an analog heart signal characteristic of the heart rhythm, according to which the heart signal is recorded, said signal is digitized and an RR series is built, **characterized in that** the RR series is automatically processed in real-time, over the course of the construction of that series, implementing the frequency processing method set out in any one of claims 1 to 7.

9. The method according to claim 8, **characterized in that** the analog heart signal is a fetal heart signal.

10. A system for the real-time acquisition and processing of a heart signal, said system comprising acquisition means (1/11) allowing the acquisition of a heart signal, first electronic means (2.4/12) making it possible to build, automatically, an RR series made up of a plurality of samples ($RR_1$) respectively defining the time intervals that separate two successive heartbeats, **characterized in that** it further comprises second electronic means (5/15) designed to process the RR series delivered by the first electronic means in accordance with the frequency processing method set out in any one of claims 1 to 7.

11. The system according to claim 10, **characterized in that** the acquisition means (11) are designed to allow the acquisition of the heart signal of a fetus, and **in that** the second electronic means (15) are designed to process the RR series delivered by the first electronic means according to the frequency processing method set out in one of claims 5 to 7.

12. The system according to claim 11, **characterized in that** it constitutes a cardiotocograph incorporating a function for measuring fetal distress.

FIG.1

FIG.2

EP 1 513 444 B1

FIG.3

FIG.4

FIG.5

16

FIG.6

# RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **LACROIX D ; LOGIER R. ; KACET S. ; HAZARD J-R ; DAGANO J.** Effects of consécutive administration of central and peripheral anticholinergic agents on respiratory sinus arrhythmia in normal subjects. *J. of the Autonomic Nervous System,* 1992, vol. 39, 211-218